# EUROPEAN PATENT APPLICATION

(11) **EP 4 752 138 A1**
(43) Date of publication of application: **03.06.2026**
(21) Application number: 24216191.7
(22) Date of filing: 28.11.2024
(51) Int. Cl.: C07D 311/76, C07C 69/738

(54) **PROCESS TO OBTAIN 8-SUBSTITUTED ISOCHROMAN-3-ONES AS INTERMEDIATES IN THE PREPARATION OF FUNGICIDES**

(71) Applicant: BASF SE, 67056 Ludwigshafen am Rhein (DE)
(72) Inventor: BORATE, Kailaskumar, Navi Mumbai, Maharashtra 400705 (IN); THOMBAL, Raju, Newasa 414607 (IN); KADUSKAR, Rahul, Navi Mumbai, Maharashtra 400705 (IN); SHYMANSKA, Nataliia, 67056 Ludwigshafen am Rhein (DE); KARALKAR, Ritesh, Navi Mumbai, Maharashtra 400705 (IN); RAUT, Dhanyakumar, Navi Mumbai, Maharashtra 400705 (IN); SATKAR, Priyanka, Navi Mumbai, Maharashtra 400705 (IN); MECHE, Sandip, Navi Mumbai, Maharashtra 400705 (IN); SHINDE, Harish, Navi Mumbai, Maharashtra 400705 (IN); GOETZ, Roland, 67056 Ludwigshafen am Rhein (DE)
(74) Representative: BASF IP Association

(57) **Abstract**

The present invention relates to a process for preparation of 8-substituted isochroman-3-ones as intermediates for the preparation of substituted enol ether fungicides. The invention also relates to processes for the preparation of intermediates of 8-substituted isochroman-3-ones synthesis.

## Description

The present invention relates to a process for preparation of 8-substituted isochroman-3-ones as intermediates for the preparation of substituted enol ether fungicides. The invention also relates to processes for the preparation of intermediates of 8-substituted isochroman-3-ones synthesis.

Enol ether fungicides are known from WO2021/153754, WO2021/219386, WO2021/219387, WO2021/219388, WO2021/219390, WO2021/249928, WO2022/008303, WO2022/013009, WO2022//03390, WO2023/072670, WO2023/072671, WO2023/072672, and EP4242198. The preparation of such fungicidal compounds was described in the abovementioned references by a coupling of the intermediates of the formula Int-I 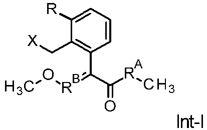 inter alia with substituted benzaldehyde oxime compounds. It has been known from IPCOM000274066D that intermediates of the type Int-I can be prepared from 8-methyl-isochroman-3-one Int-II.

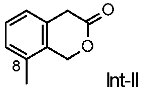

The preparation of isochroman-3-ones is known from EP0906304 B1 and involves a benzylic halogenation step. The corresponding preparation of 8-substituted isochroman-3-ones via benzylic halogenation lacks efficiency and/or suffers from selectivity issues leading to formation of side-products.

Therefore, new methods are desirable for preparing 8-substituted isochroman-3-ones and precursors thereof as synthetic intermediates for fungicides. Thus, it was an object of the present invention to overcome the disadvantages of known processes and to provide improved, more economical processes to access such compounds.

Accordingly, the present invention relates to a process for preparing compounds of formula I 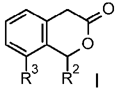 in which
R² is H or C₁-C₆-alkyl; and
R³ is C₁-C₆-alkyl, C₂-C₄-alkenyl, C₃-C₆-cycloalkyl or C₁-C₄-alkoxy wherein R³ is unsubstituted or substituted with 1, 2,
3, 4, or 5 halogens;
the process comprising reacting compounds of formula II 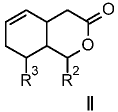
in which R² and R³ are as defined above, with an oxidizing agent.

Generally, the process is carried out at temperatures from 25°C to 300°C. A preferred temperature range is from 50°C to 180°C, more preferably from 100°C to 160°C, even more preferably at approximately 150°C.

Preferably, the reaction takes place in the presence of a solvent.

Suitable solvents are, in general, aromatic hydrocarbons such as toluene, o-, m- and p-xylene, cymene, cumene, nitrobenzene; halogenated hydrocarbons such as chlorobenzene, dichlorobenzene, dichloroethane; ethers such as dioxane, tetrahydrofuran (THF), 2-Me-THF, anisole, diphenyl ether; nitriles such as acetonitrile and propionitrile; alcohols such as ethanol, n-propanol, isopropyl alcohol, n-butanol, tert-butanol; amidic solvents such as dimethylformamide (DMF), dimethylacetamide, N-methyl-2-pyrrolidone (NMP), N-ethyl-2-pyrrolidone (NEP); sulfoxide solvents such as dimethyl sulfoxide (DMSO), sulfone solvents such as sulfolane; esters such as ethyl acetate, isopropyl acetate. It is also possible to use mixtures of the solvents mentioned. Preferred are high-boiling solvents such as xylene, cumene, chlorobenzene, dichlorobenzene, DMSO, DMF, and NMP. More preferred is diphenyl ether.

Suitable oxidizing agents are generally stoichiometric oxidants or catalytic systems comprising a metal catalyst and a terminal oxidant.

Examples of stoichiometric oxidants include but are not limited to 4,5-dichloro-3,6-dioxocyclohexa-1,4-diene-1,2-dicarbonitrile (DDQ), oxygen (O₂), chloranil, (2,2,6,6-tetramethylpiperidin-1-yl)oxyl (TEMPO), tert-butyl hydroperoxide, and sulfur. Preferably O₂ is employed as a stoichiometric oxidant. Stoichiometric oxidants are generally employed in equimolar or excess amounts with respect to the compound II. Preferably, between 1.0 and 3.0 molar equivalents of a stoichiometric oxidant are employed with respect to the compound II.

Examples of metal catalysts comprised in catalytic systems are Pd/C, Pd(OH)₂, Pd/Al₂O₃, Pd/BaSO₄, Pd/CaCO₃, Ru/C, Pt/C, Pt:Mo, Pd:Pt:Mo, PdNP. Preferably, Pd/BaSO₄ is employed as the metal catalyst. Preferably, between 0.001 and 2.0 molar equivalents of the metal catalyst are employed with respect to the compound II, more preferably between 0.01 and 0.5 molar equivalents of the metal catalyst are employed with respect to the compound II.

Examples of terminal oxidants comprised in catalytic systems are air, oxygen (O₂), or oxygen/nitrogen (O₂/N₂) mixtures. Stoichiometric oxidants as described above may also be used as terminal oxidants. Preferably O₂ is employed as a terminal oxidant. Gaseous terminal oxidants are generally employed in large excess with respect to the compound II. Non-gaseous terminal oxidants are employed in equimolar or excess amounts with respect to the compound II. More preferably, between 1 and 5 molar equivalents of a non-gaseous terminal oxidant is employed, more preferably between 1 and 3 equivalents.

The reaction period after addition of the oxidizing agent is not particularly limited and typically it is in the range of 6 to 48 h, preferably from 8 to 16 h.

Optionally, an additive is employed in the process to obtain compounds I. Suitable additives are C₂-C₁₂-alkenes and C₁-C₁₂-aldehydes which are unsubstituted or substituted with one phenyl ring. Further suitable additives are benzenes that are substituted with one nitro (NO₂) group and optionally substituted with one further substituent selected from alkyl substituents. Preferably, nitrobenzene (C₆H₅NO₂) is employed as an additive. The additive may be employed in substoichiometric, equimolar, or excess amounts with regard to the amount of compound II used. Preferably, between 0.1 and 2.0 equivalents of the additive are employed with regard to the compound II. More preferably, between 0.3 and 0.7 molar equivalents of the additive are employed.

Further, the present invention relates to a process for preparing compounds I, wherein the compounds of formula II are obtained by reacting compounds of formula III 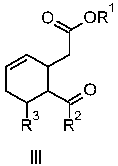 in which
R¹ is H, C₁-C₁₂-alkyl, phenyl, C₁-C₄-alkyl-phenyl, or C₁-C₄-alkyl-C₃-C₆-cycloalkyl, wherein phenyl rings are unsubstituted or substituted with one halogen;
and R² and R³ are as defined above; with a reducing agent.

Generally, the process to obtain compounds II is carried out at temperatures from -10°C to 120°C. A preferred temperature range is from 0°C to 100°C, more preferably from 0°C to 60°C, even more preferably from 0°C to 25°C.

Preferably, the reaction takes place in presence of a solvent.

Suitable solvents are, in general, aromatic hydrocarbons such as toluene, o-, m- and p-xylene, cymene, cumene; halogenated hydrocarbons such as chlorobenzene, dichlorobenzene, dichloromethane, dichloroethane; ethers such as dioxane, THF, 2-Me-THF, cyclopentyl methyl ether, diisopropyl ether, diphenyl ether; alcohols such as methanol, ethanol, n-propanol, isopropyl alcohol, n-butanol, tert-butanol; water and aqueous solutions of inorganic bases such as sodium hydroxide, potassium hydroxide, sodium carbonate, potassium carbonate. It is also possible to use mixtures of the solvents mentioned. Preferred are ether and alcohol solvents. More preferred are ethanol, methanol, THF. Even more preferred is ethanol.

Suitable reducing agents are, in general, stoichiometric reductants such as NaBH₄, NaCNBH₃, LiAlH₄, LiBH₄, Et₃SiH/trifluoroacetic acid (TFA), Sml₂, and metal borides, such as Nickel/Cobalt borides. NaBH₄ can be used alone or in combination with a Lewis acid (for example, NaBH₄/CeCl₃) or in combination with a base (for example, NaBH₄/NaOH). Further suitable reducing agents comprise a metal catalyst in the presence of a terminal reductant such as hydrogen (H₂), for example as described in Pietropaolo et al, Appl. Catal. A. Gen. 2010 p. 77 and references therein, or transfer hydrogenations such as those using Pd/C, NEt₃/HCO₂H. Preferably NaBH₄ or NaCNBH₃ are employed, more preferably NaBH₄ is employed.

The reducing agent may be employed as is or in solution. The reducing agent may be employed in stoichiometric amounts or in excess relative to the compound III. Preferably between 1.0 and 3.0 equivalents are employed relative to compound III, more preferably 1.5 equivalents are employed relative to compound III.

The reaction period after addition of the reducing agent is not particularly limited and typically it is in the range of 30 min to 6 h, preferably from 1 h to 3 h.

Optionally, the reaction can be stopped by quenching. Suitable quenching agents are, in general, aqueous solutions such as aqueous hydrochloric acid or aqueous ammonium chloride, preferably aqueous hydrochloric acid.

Further, the present invention relates to a process to obtain compounds I, wherein compounds of formula III are obtained by a process comprising reacting compounds IV 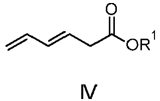 in which R¹ is as defined above; with compounds V 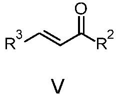 in which R² and R³ are as defined above, in the presence of an additive.

Generally, the process is carried out at temperatures from -80°C to 150°C, more preferably from -20°C to 30°C.

Preferably, the reaction to prepare compounds III takes place in the presence of a solvent.

Suitable solvents are, in general, aromatic hydrocarbons such as toluene, o-, m- and p-xylene, cymene, cumene; halogenated hydrocarbons such as chlorobenzene, dichlorobenzene, dichloromethane, dichloroethane; ethers such as diethyl ether, dioxane, THF, 2-Me-THF, cyclopentyl methyl ether, diisopropyl ether, diphenyl ether; nitriles such as acetonitrile and propionitrile; alcohols such as methanol, ethanol, n-propanol, isopropyl alcohol, n-butanol, tert-butanol; amidic solvents such as DMF, dimethyl acetamide, NMP, NEP; sulfoxide solvents such as DMSO, sulfone solvents such as sulfolane; esters such as ethyl acetate, isopropyl acetate. It is also possible to use mixtures of the solvents mentioned. Preferred are halogenated hydrocarbons and ether solvents. More preferred are DCM, dichloroethane, and diethyl ether. Even more preferred is DCM.

The amount of compound V relative to compound IV is preferably in the range from 1.0 to 5.0 molar equivalents, more preferably 1.0 to 3.0 molar equivalents, even more preferably from 1.0 to 1.5 molar equivalents.

Suitable additives are, in general, Lewis acids such as boron trifluoride diethyl etherate, boron trifluoride gas, SnCl₄, ZnCl₂, MgCl₂, CoCl₂, Zn(OTf)₃, CuCl₂, Cu(OTf)₂, Cu(BF₄)₂, Sc(OTf)₃, BiCl₃, LiCl, AgBF₄, AlCl₃, Me₂AlCl; Bronsted acids such as p-toluenesulfonic acid, phosphoric acid; secondary amines such as diethylamine, pyrrolidine, piperidine, proline, morpholine; tertiary amines such as trimethylamine, triethylamine, tributylamine, diisopropylethylamine; nitrogen-containing heteroaromatics such as pyridine, collidine, lutidine, 4-(N,N-dimethyl)-aminopyridine; amidines such as 1,8-diazabicyclo[5.4.0]undec-7-ene (DBU), 1,5-diazabicyclo[4.3.0]non-5-ene (DBN); and bicyclic amines such as 1,4-diazabicyclo[2.2.2]octane (DABCO); inorganic salts such as NaF, KF, LiF; ionic liquids such as imidazolium salts; alkali metal and alkaline earth metal phosphate catalysts such as lithium phosphate, sodium phosphate, potassium phosphate, calcium phosphate.

The amount of additive relative to compound IV is preferably in the range from 0.01 to 3.0 equivalents, more preferably from 0.05 to 2.0 equivalents, even more preferably from 0.1 to 1.2 equivalents.

Compounds IV and V are commercially available or can be obtained according to methods described in the literature (e.g., T. R. Hoye et al., Synthetic Communications 1982, 12, 183-187 and K. S. Kim et al., Synthesis 1987, 11, 1017-1018).

The reaction mixtures are worked up in a customary manner, for example by mixing with water, separating the phases and, if appropriate, chromatographic purification of the crude products. In some cases, the intermediates and end products are obtained in the form of colorless or slightly viscous oils which can be freed from volatile components or purified under reduced pressure and at moderately elevated temperatures. If the intermediates and end products are obtained as solids, purification can also be carried out by recrystallization or digestion.

In addition, the invention also relates to novel compounds of formula I 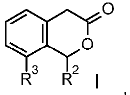 compounds of formula II 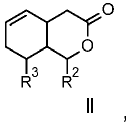 and compounds of formula III 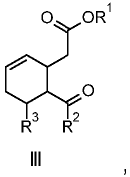 in which R¹, R², and R³ are defined as defined herein.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. It is to be understood that the term "comprising" is not limiting. For the purposes of the present invention the term "consisting of" is considered to be a preferred embodiment of the term "comprising".

Unless otherwise indicated, the following definitions are set forth to illustrate and define the meaning and scope of the various terms used to describe the invention herein and the appended claims. These definitions should not be interpreted in the literal sense as they are not intended to be general definitions and are relevant only for this application.

The term "compounds I" refers to compounds of formula I. Likewise, this terminology applies to all sub-formulae, e. g. "compounds II" refers to compounds of formula II or "compounds V" refers to compounds of formula V, etc.

The term "independently" when used in the context of selection of substituents for a variable, it means that where more than one substituent is selected from a number of possible substituents, those substituents may be the same or different.

The organic moieties or groups mentioned in the above definitions of the variables are collective terms for individual listings of the individual group members. The term "Cᵥ-C_{w}" indicates the number of carbon atom possible in each case.

The term "halogen" refers to fluorine, chlorine, bromine and iodine.

The term "C₁-C₁₂-alkyl" refers to a straight-chained or branched saturated hydrocarbon group having 1 to 12 carbon atoms, for example, methyl (CH₃), ethyl (C₂H₅), propyl, 1-methylethyl (isopropyl), butyl, 1-methylpropyl, 2-methylpropyl, 1,1-dimethylethyl, heptyl, decanyl, dodecanyl.

The term "C₂-C₄-alkenyl" refers to a straight-chain or branched unsaturated hydrocarbon radical having 2 to 4 carbon atoms and a double bond in any position such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₁-C₄-alkoxy" refers to a straight-chain or branched alkyl group having 1 to 4 carbon atoms which is bonded via an oxygen, at any position in the alkyl group, e.g. OCH₃, OCH₂CH₃, O(CH₂)₂CH₃, 1-methylethoxy, O(CH₂)₃CH₃, 1-methylpropoxy, 2-methylpropoxy or 1,1-dimethylethoxy.

The term "C₃-C₆-cycloalkyl" refers to monocyclic saturated hydrocarbon radicals having 3 to 6 carbon ring members, such as cyclopropyl (C₃H₅), cyclobutyl, cyclopentyl or cyclohexyl.

The term "C₁-C₄-alkyl-phenyl" refers to a straight-chained or branched saturated hydrocarbon group having 1 to 4 carbon atoms and carrying a phenyl substituent, for example, benzyl (-CH₂-C₆H₅), homobenzyl (-CH₂CH₂-C₆H₅), 1-phenylethyl (-CH(CH₃)-C₆H₅).

The term "C₁-C₄-alkyl-C₃-C₆-cycloalkyl" refers to a straight-chained or branched saturated hydrocarbon group having 1 to 4 carbon atoms and carrying a C₃-C₆-cycloalkyl substituent, for example, -CH₂-cyclopropyl, -CH₂CH₂-cyclobutyl, -CH₂-cyclohexyl.

In respect of the variables, the embodiments of the compounds II, III, IV and V correspond to the embodiments of the compounds I.

Preference is given to those compounds I and where applicable also to compounds II, III, IV and V, wherein the substituents and variables (such as R¹, R², R³) have independently of each other or more preferably in combination (any possible combination of 2 or more substituents as defined herein) the following meanings:
According to one embodiment, the substituent R¹ is unsubstituted C₁-C₆-alkyl, more preferably methyl or ethyl, in particular ethyl.
Preferably, R² is H.
Preferably, R³ is unsubstituted C₁-C₆-alkyl, more preferably methyl or ethyl, in particular methyl.
According to one embodiment, the substituent R¹ in formula III is unsubstituted C₁-C₆-alkyl, more preferably methyl or ethyl, in particular ethyl.
Preference is given to those compounds I, II, and III, wherein the substituents R² and R³ have independently of each other or more preferably in combination (any possible combination of 2 or more substituents as defined herein) the following meanings:
   According to one embodiment, R² is H or C₁-C₃-alkyl, more preferably H or methyl, in particular H.
   According to one embodiment, R³ is unsubstituted C₁-C₆-alkyl, more preferably methyl or ethyl, in particular ethyl.

Preferably, compound I is 8-methylisochroman-3-one:

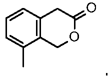

Preferably, compound II is 8-methyl-1,4,4a,7,8,8a-hexahydroisochromen-3-one:

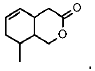

Preferably, compound III is ethyl 2-(6-formyl-5-methyl-cyclohex-2-en-1-yl) acetate:

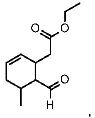

Further, the invention relates to the use of compounds II for the preparation of compounds I.

Further, the invention relates to the use of compounds III for the preparation of compounds I.

### Examples

### Example 1: Preparation of 8-methylisochroman-3-one

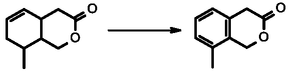

To a magnetically stirred solution of the 8-methyl-1,4,4a,7,8,8a-hexahydroisochromen-3-one (2 g, 12.03 mmol) and Pd/BaSO₄ (200 mg, 10 wt%) in diphenyl ether (10 mL) was added nitrobenzene (741 mg, 6.01 mmol, 50 mol%). The reaction mixture was heated at 150°C under oxygen atmosphere until consumption of starting material. After the completion of reaction, the reaction mixture was cooled to room temperature (RT), filtered through celite and celite bed was washed with methanol (MeOH). The combined organic layers were concentrated under reduced pressure and purified over a silica gel to furnish 8-methylisochroman-3-one (1.08 g, 55% yield).

¹H NMR (300 MHz, CDCl₃): δ 7.28 - 7.24 (q, *J =* 12 Hz, 1H), 7.15 - 7.13 (d, *J =* 6.0 Hz, 1H), 7.08 - 7.05 (d, *J =* 9.0 Hz, 1H), 5.37 (s, 2H), 3.70 (s, 2H), 2.34 (s, 3H).

### GCMS Method

| | | | | |
|---|---|---|---|---|
| Total Flow: | 35.4 mL/min | Oven Temp. Program: | | |
| Column Flow: | 1.04 mL/min | Rate (°C/min) | Temp. (°C) | Hold Time (min) |
| Linear Velocity: | 36.8 cm/sec | - | 40.0 | 2.00 |
| Purge Flow: | 3.0 mL/min | 20.00 | 250.0 | 0.00 |
| Split Ratio: | 30.0 | 50.00 | 280.0 | 2.00 |
| Column: | Rxi-5Sil MS, 30m, 0.25mm ID, 0.25µm | | | |

8-Methyl-1,4,4a,7,8,8a-hexahydroisochromen-3-one as a mixture of diastereoisomers: 10.40 & 10.57 min.

8-Methylisochroman-3-one: 10.81min.

### Example 2: Preparation of 8-methyl-1,4,4a,7,8,8a-hexahydroisochromen-3-one

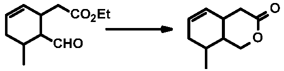

Ethyl 2-(6-formyl-5-methyl-cyclohex-2-en-1-yl) acetate (228 g, 0.952 mol) and MeOH (2000 mL) were mixed at 25°C. The reaction mixture was cooled down to 0°C and a solution of NaBH₄ (54.09 g 1.43 mol) in NaOH (1 N, 1240 mL) was added dropwise over 60 min. The reaction mass was further stirred at 0°C for 1.5 h. After consumption of starting material, the reaction mixture was quenched with HCl (1 N, 2700 ml) to adjust the pH to 3-4 at 0°C (Tr). After quenching, the reaction mixture was warmed to RT and extracted with DCM (2 x 1000 mL). The organic layer was washed with brine (500 ml), dried over sodium sulfate, and evaporated to obtain 8-methyl-1,4,4a,7,8,8a-hexahydroisochromen-3-one (135 g, 85% yield).
m/z by GCMS 166

### GCMS Method

| | | | | |
|---|---|---|---|---|
| Total Flow: | 35.4 mL/min | Oven Temp. Program: | | |
| Column Flow: | 1.04 mL/min | Rate (°C/min) | Temp. (°C) | Hold Time (min) |
| Linear Velocity: | 36.8 cm/sec | - | 40.0 | 2.00 |
| Purge Flow: | 3.0 mL/min | 20.00 | 250.0 | 0.00 |
| Split Ratio: | 30.0 | 50.00 | 280.0 | 2.00 |
| Column: | Rxi-5Sil MS, 30m, 0.25mm ID, 0.25µm | | | |

Ethyl 2-(6-formyl-5-methyl-cyclohex-2-en-1-yl) acetate as mixture of diastereoisomers: 10.17 & 10.25 min 8-Methyl-1,4,4a,7,8,8a-hexahydroisochromen-3-one as mixture of diastereoisomers: 10.40 & 10.57 min, m/z 166.

### Example 3: Preparation of ethyl 2-(6-formyl-5-methyl-cyclohex-2-en-1-yl) acetate

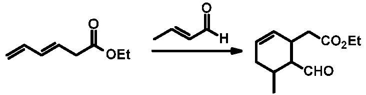

DCM (1500 mL) was charged into the ethyl (3E)-3,5-hexadienoate (502 g, 3.587 mol) in the 20 L reactor followed by the addition of crotonaldehyde (301.76 g, 4.305 mol). The reaction mixture was cooled to 0°C and 50% boron trifluoride diethyl etherate solution in diethyl ether (254.31 g, 0.896 mol) solution diluted with DCM (1000 mL) added dropwise over approximately 60 min at 0°C. After completion of addition, the reaction mixture was further stirred reaction at 25°C for 16 h. After completion of the reaction, the reaction mass was quenched using saturated solution of sodium bicarbonate at 0°C within 40 min. Separated the organic phase and aqueous layer was extracted using dichloromethane (500 mL). Organic layer was washed with brine (500 ml), dried over sodium sulphate, and evaporated to obtain ethyl 2-(6-formyl-5-methyl-cyclohex-2-en-1-yl) acetate (640 g; 85% yield).

¹H NMR (500 MHz, Chloroform-*d*) δ 9.89 - 9.52 (m, 1H), 5.84 - 5.47 (m, 2H), 4.14 (qd, *J* = 7.1, 1.3 Hz, 3H), 3.04 - 2.92 (m, 1H), 2.55 - 2.37 (m, 3H), 2.36 - 2.13 (m, 3H), 1.90 (dddt, *J* = 15.5, 5.9, 4.1, 2.3 Hz, 1H), 1.76 (dddd, *J =* 18.1, 7.4, 3.6, 2.0 Hz, 1H), 1.31 - 1.21 (m, 3H), 1.05 (dd, *J* = 19.2, 6.6 Hz, 4H).

### GCMS Method

| | | | | |
|---|---|---|---|---|
| Total Flow: | 35.4 mL/min | Oven Temp. Program: | | |
| Column Flow: | 1.04 mL/min | Rate (°C/min) | Temp. (°C) | Hold Time (min) |
| Linear Velocity: | 36.8 cm/sec | - | 40.0 | 2.00 |
| Purge Flow: | 3.0 mL/min | 20.00 | 250.0 | 0.00 |
| Split Ratio: | 30.0 | 50.00 | 280.0 | 2.00 |
| Column: | Rxi-5Sil MS, 30m, 0.25mm ID, 0.25µm | | | |

Ethyl (3E)-3,5-hexadienoate: 6.38min.

Ethyl 2-(6-formyl-5-methyl-cyclohex-2-en-1-yl) acetate as a mixture of diastereoisomers: 10.17 & 10.25 min.

## Claims

1. A process for preparing compounds of formula I 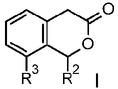 in which
R² is H or C₁-C₆-alkyl; and
R³ is C₁-C₆-alkyl, C₂-C₄-alkenyl, C₃-C₆-cycloalkyl or C₁-C₄-alkoxy wherein R³ is unsubstituted or substituted with 1, 2, 3, 4, or 5 halogens;
the process comprising reacting compounds of formula II 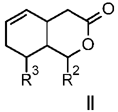
in which R² and R³ are as defined above,
with an oxidizing agent.

2. The process of claim 1, in which the oxidizing agent comprises Pd/BaSO₄ and O₂.

3. The process of any of the claims 1 to 2, in which R² is H.

4. The process of any of the claims 1 to 3, in which R³ is methyl.

5. The process of any of the claims 1 to 4, wherein the compounds of formula II are obtained by reacting compounds of formula III 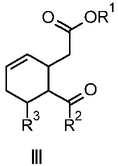 in which
R¹ is H, C₁-C₁₂-alkyl, phenyl, C₁-C₄-alkyl-phenyl, or C₁-C₄-alkyl-C₃-C₆-cycloalkyl, wherein phenyl rings are unsubstituted or substituted with one halogen; and
R² and R³ are as defined in any of the claims 1, 3, and 4;
with a reducing agent.

6. The process of claim 5, in which the reducing agent is NaBH₄.

7. The process of any of the claims 5 to 6, in which R¹ is ethyl.

8. The process of any of the claims 5 to 7, wherein the compounds of formula III are obtained by a process comprising reacting compounds of formula IV 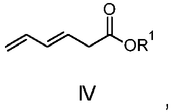 in which R¹ is as defined in claim 5 or 7; with compounds of formula V 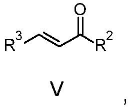 in which R² and R³ are as defined in claim 1, 3, or 4; in the presence of an additive.

9. The process of claim 8, in which the additive is boron trifluoride diethyl etherate.

10. Compounds of formula I 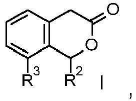 compounds of formula II 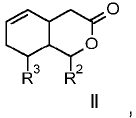 and compounds of formula III 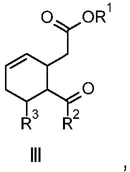 in which R¹ is defined as in claim 5 and R² and R³ are defined as in claim 1.

11. Compounds of formula I, compounds of formula II, and compounds of formula III according to claim 10, wherein R² is H.

12. Compounds of formula I, compounds of formula II, and compounds of formula III according to any of the claims 10 to 11, wherein R³ is methyl.

13. Compounds of formula III according to any of the claims 10 to 12, wherein R¹ is ethyl.

14. Use of compounds of formula II 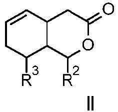 or compounds of formula III 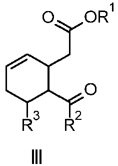 for the preparation of compounds of formula I 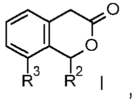 in which R¹ is defined as in claim 5 and R² and R³ are as defined as in any of the claim 1, 3, and 4.
